# EUROPEAN PATENT SPECIFICATION

(11) **EP 3 157 444 B1**
(45) Date of publication and mention of the grant of the patent: **09.12.2020**
(21) Application number: 15747537.7
(22) Date of filing: 19.06.2015
(51) Int. Cl.: A61B 17/132, A61B 17/135

(54) **AUTOMATIC BLOOD FLOW CONTROL SYSTEM AND TOURNIQUET**
SYSTEM ZUR AUTOMATISCHEN BLUTFLUSSSTEUERUNG UND TOURNIQUET
SYSTÈME DE RÉGULATION DU DÉBIT SANGUIN AUTOMATIQUE ET GARROT

(30) Priority: 20.06.2014 PL 40861914
(43) Date of publication of application: 26.04.2017
(73) Proprietor: Instytut Kardiologii Im. Prymasa Tysiaclecia, 04-628 Warszawa (PL)
(72) Inventor: KRUK, Mariusz, 05-070 Sulejowek (PL)
(74) Representative: Patpol Kancelaria Patentowa Sp. z o.o.
(86) International application number: PCT/IB2015/054621
(87) International publication number: WO 2015/193847

(56) References cited:
- EP-A2- 0 462 088
- WO-A1-2014/027347
- WO-A2-2008/070164
- TW-U- M 447 209
- US-A1- 2007 179 521
- US-A1- 2014 024 986

## Description

### Technical field

The subject of the invention is a tourniquet and an automatic blood flow control system comprising it. More particularly, the invention relates to dressing blood vessels, especially arteries damaged for the purpose of endovascular procedures.

### Prior art

Prior art discloses compression bandages, applicable when removing the arterial sheath after a cardiac catheterization - angiography, catheter ablation, or after other procedures that require reaching blood pressure, i.e., generally endovascular procedures. These include a tourniquet known from Polish patent application No. P.396805 and International Patent Publication No. WO2013060883.

These previously known solutions, capable of achieving the desired homeostasis often require implementation of a series of consecutive and successive actions, and often cooperation of at least two people during and after the procedure - usually a doctor and a nurse. These activities include putting a tourniquet on a limb, fastening the tourniquet on the limb, pressing the infusion site during the removal of the cannula, and fixing the tourniquet in such a way as to maintain the appropriate compression on the infusion site and periodic adjustment of pressure decreasing it progressively, until the tourniquet is finally removed. Pressing force should be selected so as to facilitate meeting two conditions at the same time: hemostasis, *i.e*., no blood flow and maintaining blood flow in the pressed vessel, achieved when the pressure exerted by the bandage is equal to or lower than the blood pressure. Since blood pressure is subject to cyclical changes, fulfilment of this condition is possible, if a pressure corresponding to systolic pressure is ensured. Using too much pressure may cause crushing the vessel, resulting in loss of blood flow, resulting in the potential permanent damage due to thrombosis, obstruction and even a possible limb ischemia.

The systolic blood pressure varies in time after the procedure. Maintaining the proper, not too high and not too low, pressure in the postoperative phase requires frequent adjustment by the qualified personnel.

Prior art discloses known numerous devices for automatic control of pressure applied to the limb. These devices are used in automatic measurement of patients' blood pressure. An example of such a device is disclosed in U.S. Patent No. 5660182. It discloses a system equipped with a CPU, which is connected with a pressure sensor, microphone and switches by means of the filters and analogue/digital converters. The CPU is connected to a memory, output means and an interface device adapted to set a pressure regulating valve, a diverter valve and air pump via the data bus. The diverter valve allows to supply air from the pump to the sleeve put on the patient's arm or discharge air therefrom.

Taiwanese utility model No. TWM447209Y discloses a device to control bleeding with electronic pressure control. This device employs electronic control of the pressure exerted by a tourniquet at the site of bleeding, according to the results of blood pressure measurement performed using an independent device. Such a solution provides the ability of adaptive customization of the compression of the tourniquet to the changes in patient's pressure after the surgery. As a result, the damaged vessel is constantly subjected to compression slightly greater than systolic blood pressure and thus allowing to control the bleeding. At the same time the vessel is not subjected to excessive compression risking in its capping.

The US Patent No. 4,321,929 discloses an automatic blood control device, wherein the compression of the bandage is applied through means of the balloon inflated to a predetermined pressure exceeding the systolic blood pressure, to be measured by means of a separate sensor, under the control of the microprocessor.

WO 2014/027347 discloses a vessel pressure control device configured for selectively applying pressure to a radial artery, which comprises a sensor configured to sense one or more parameters associated with blood flow through a portion of the radial artery, a pressure application element adapted to apply variable amounts of pressure to the portion of the radial artery, and a controller configured to apply logic and generate a signal to control the pressure application element in response to the one or more measured parameters. The compression of the device is adjusted, so that while preventing bleeding form the radial artery, at least some blood flow is maintained through the radial artery portion in an amount sufficient to reduce or prevent radial artery occlusion.

Prior art, ACC Cardiovasc Interv 2010;3:1022-1031, Eur Heart J 2012;33:2521-2528, discloses a method of using a tourniquet equipped with a compression element in the form of a balloon filled with gas under pressure. In the method, first apply the pressure in the balloon substantially higher than the patient's systolic blood pressure and then gradually reduce it with the constant visual inspection. In the event of loss of blood, increase the pressure until it ceases. Such a solution requires a significant effort of staff and is associated with a risk of leading to loss of blood through too fast loosening of compression on the damaged vessel.

The aim of the present invention is to solve the above-mentioned problems.

### The gist of the invention

The present invention is disclosed in the appended set of claims. The present invention provides a tourniquet having a controlled compression element adapted to compress the damaged blood vessel at the lesion site, a blood flow sensor for detecting the blood flow through the compressed vessel and a blood loss sensor for detecting the blood flow from the compressed vessel, wherein the said tourniquet is further provided with a second compression element configured to compress a healthy vessel and control the blood flow therein, wherein the first and the second compression elements are controlled independently.

An automatic compression bandage system is also provided herein, wherein said system comprises the tourniquet of the invention, an interface for electronic pressure control of compression elements and a CPU provided with a memory and connected to the compression element interface. Preferably, the compression element of the tourniquet of the invention is a balloon, wherein the system further comprises a pump and an automatic valve whose control input is connected to the CPU and adapted to connect the balloon to the pump and to empty it according to the setting of its control input.

Also preferably both compression elements are balloons and the first balloon and the second balloon can be pumped and deflated independently of each other. More preferably, the blood flow sensor for detecting the blood flow through the compressed vessel and the blood loss sensor for detecting the detecting the blood flow through the compressed vessel and means adapted to detect the blood flow from compressed vessel.

Means for detecting the blood flow through the compressed vessel and means adapted to detect the blood flow are preferably located in the immediate vicinity of the compression means. As a result, the system can be implemented as a single, compact device. This is possible because the detection of blood flow is possible, despite the vessel being compressed. In such conditions - for example - precise measurement of systolic and diastolic pressure is not possible.

The compression element is preferably a balloon, whereas the system further comprises a pump and an automatic valve whose control input is connected to a CPU adapted to connect the balloon to the pump and empty it according to the setting of its control input.

Means adapted to detect the blood flow are preferably a heart rate monitor, a microphone, a pressure sensor or a Doppler probe or a plethysmograph whose output is connected to the input of the CPU capable of detecting changes in the indicated value and/or analyzing the signal supplied.

Preferably, the compression system comprises a second compression element configured to compress a healthy vessel and control the blood flow therein.

The tourniquet according to the invention is provided with a controlled compression element and comprises a blood flow sensor, and the blood loss sensor. The compression element is preferably a balloon, whereas the system further comprises a pump and an automatic valve whose control input is connected to a CPU adapted to connect the balloon to the pump and to empty it according to the setting of its control input. Means adapted to detect the blood flow are preferably a heart rate monitor, a microphone, a pressure sensor or a Doppler probe or a plethysmograph connected to a device capable of detecting changes in the output signal.

The advantage of the invention is to automate the compression of the punctured vessel, in a manner ensuring both hemostasis and flow in the compressed vessel, while continuously monitoring the status of homeostasis and patency of the vessel.

The further advantage of the invention is that it allows compensation of a blood flow decrease or even forcing an increased blood flow in the vessel from which the bleeding is obstructed - by compressing a healthy vessel. Paradoxically by increasing the blood flow through the healing vessel the risk of complications can be reduced. Without the ability to automatically adjust the compression on both vessels and automatic response to limb ischemia and pressure changes in the healing vessel such a blood flow increase would have a high risk for the patient.

### Description of the figures

The invention is described in the embodiments in the drawings, in which: Fig. 1a shows a tourniquet according to the embodiment of the invention put on the wrist, Fig. 1b shows a tourniquet according to another embodiment put on the wrist, Fig 2a shows a block diagram of the embodiment of a system according to the invention, Fig. 2b shows a block diagram of an alternative embodiment of the system according to the invention, Fig. 2c shows a block diagram of yet another embodiment of the system according to the invention Fig. 3 shows a flowchart of a method according to the invention, and Fig. 4 shows the measurement of blood flow through a vessel using a plethysmograph.

blood flow from the compressed vessel are located in the immediate vicinity of the compression element.

Described herein is a method for automatic control of blood from the damaged vessel, which is used in a dressing system provided with a tourniquet, a bandage and the first compression element. The method comprises the stage of applying pressure exceeding the systolic blood pressure of the patient via the compression element to a lesion site, so that the blood stops flowing through the damaged vessel, then the stage of reducing the pressure. In the pressure reduction stage, the pressure is reduced by a predetermined drop value, wherein if no blood flow is detected in the damaged vessel, then pressure reduction stage is repeated and if blood flow in the damaged vessel is detected, the pressure reduction stage is interrupted for a pressure drop time. Preferably, after each pressure reduction, pressurization stage is additionally performed when blood flow from the damaged vessel is detected. Execution of the method is interrupted when the pressure exerted by the compression element drops below the patient's diastolic pressure, while no loss of blood is detected. Blood flow through the vessel is preferably established on the basis of the signal from the pressure meter or microphone or Doppler probe or plethysmograph. Plethysmograph measurement can be used at the end of the limb, particularly on the digit. Digits are supplied with blood by parallel blood vessels, in particular parallel arteries. Accordingly, during the plethysmograph measurement, parallel vessel compression is preferably used so that the flow or no flow detected by the plethysmograph is connected with the flow in the damaged vessel. Preferably, the flow in the parallel vessel is also subject to monitoring and compression on this vessel is reduced when no flow is detected.

The automatic compression bandage system according to the invention is provided with a tourniquet with a compression element adapted to compress the damaged blood vessel at the lesion site, provided with an interface for electronic pressure control, and a CPU provided with a memory, connected to the compression element interface. The system is further provided with means for detecting the blood flow through the compressed vessel and means adapted to detect the blood flow from compressed vessel.

Means for detecting the blood flow through the compressed vessel and means adapted to detect the blood flow are preferably located in the immediate vicinity of the compression means. As a result, the system can be implemented as a single, compact device. This is possible because the detection of blood flow is possible, despite the vessel being compressed. In such conditions - for example - precise measurement of systolic and diastolic pressure is not possible.

The compression element is preferably a balloon, whereas the system further comprises a pump and an automatic valve whose control input is connected to a CPU adapted to connect the balloon to the pump and empty it according to the setting of its control input.

Means adapted to detect the blood flow are preferably a heart rate monitor, a microphone, a pressure sensor or a Doppler probe or a plethysmograph whose output is connected to the input of the CPU capable of detecting changes in the indicated value and/or analyzing the signal supplied.

The compression system of the invention comprises a second compression element configured to compress a healthy vessel and control the blood flow therein.

The tourniquet according to the invention is provided with a controlled compression element and comprises a blood flow sensor, and the blood loss sensor. The compression element is preferably a balloon, whereas the system further comprises a pump and an automatic valve whose control input is connected to a CPU adapted to connect the balloon to the pump and to empty it according to the setting of its control input. Means adapted to detect the blood flow are preferably a heart rate monitor, a microphone, a pressure sensor or a Doppler probe or a plethysmograph connected to a device capable of detecting changes in the output signal.

The advantage of the invention is to automate the compression of the punctured vessel, in a manner ensuring both hemostasis and flow in the compressed vessel, while continuously monitoring the status of homeostasis and patency of the vessel.

The further advantage of the invention is that it allows compensation of a blood flow decrease or even forcing an increased blood flow in the vessel from which the bleeding is obstructed - by compressing a healthy vessel. Paradoxically by increasing the blood flow through the healing vessel the risk of complications can be reduced. Without the ability to automatically adjust the compression on both vessels and automatic response to limb ischemia and pressure changes in the healing vessel such a blood flow increase would have a high risk for the patient.

### Description of the figures

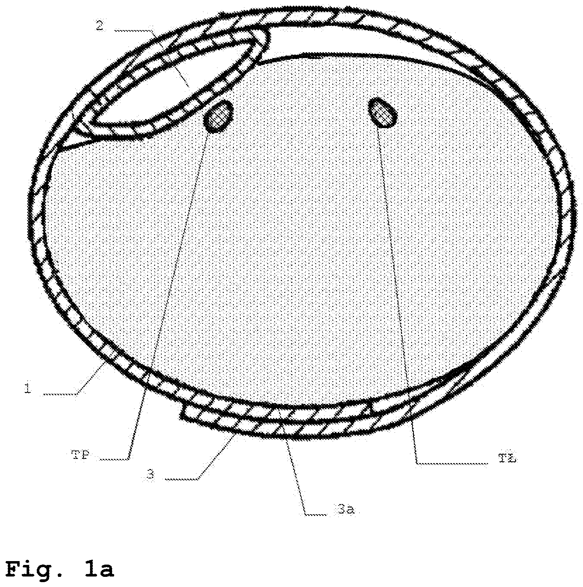

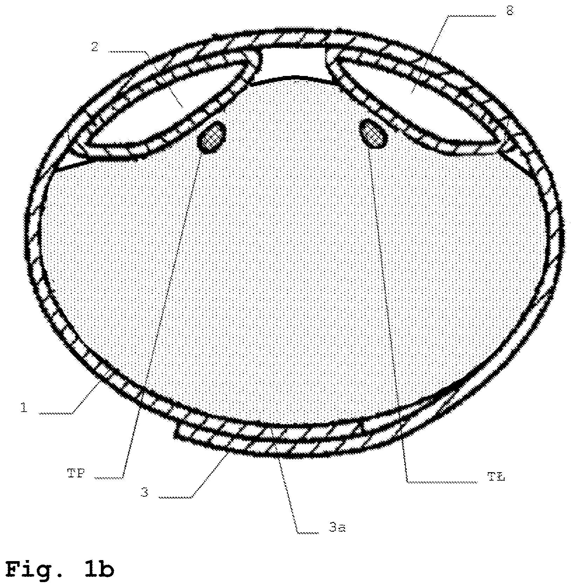

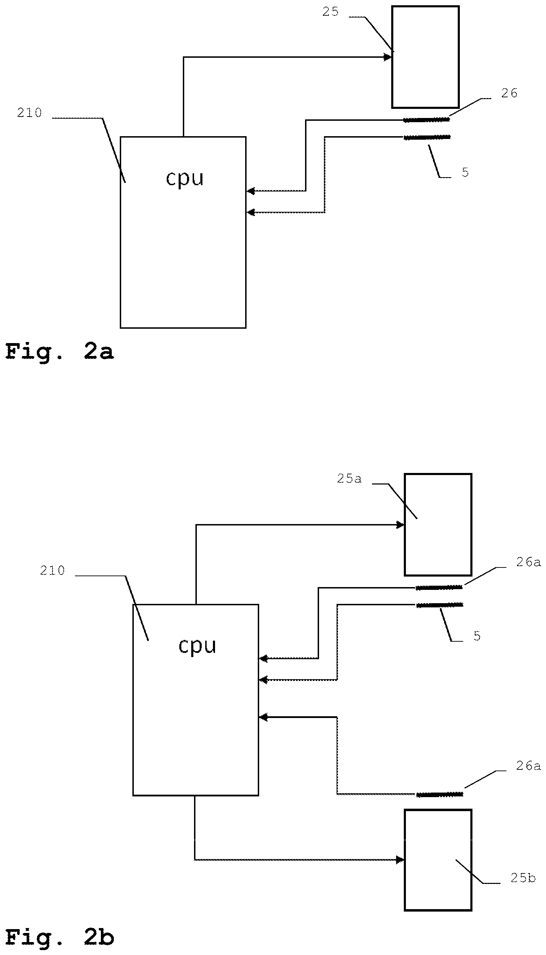

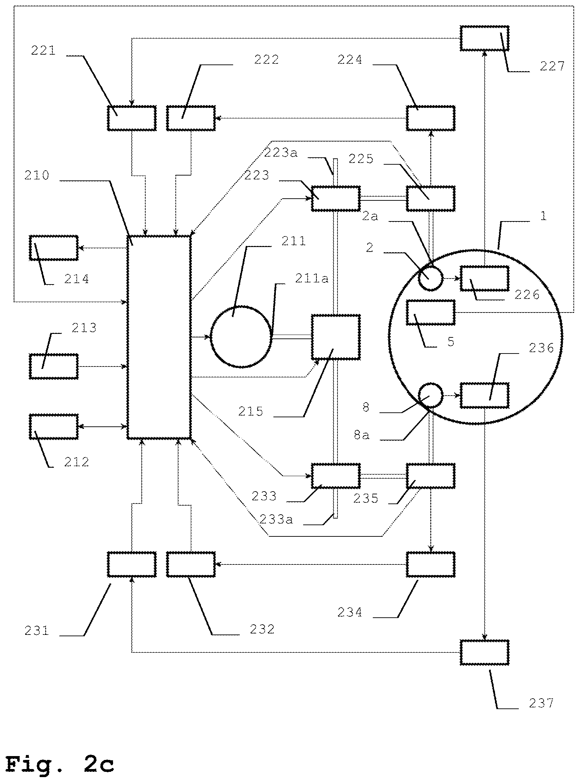

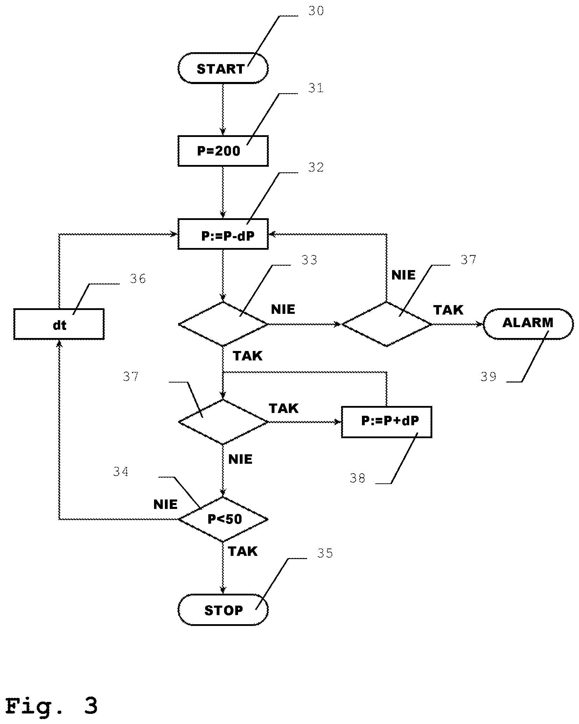

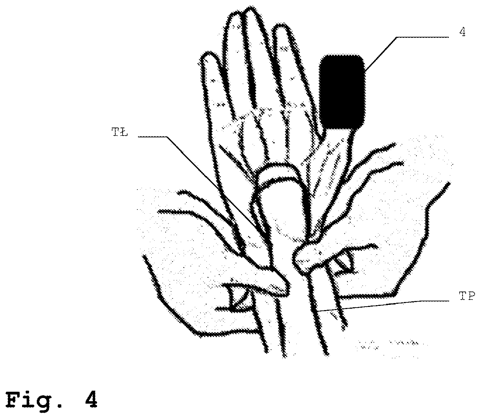

The invention is described in the embodiments in the drawings, in which: Fig. 1a shows a tourniquet according to the embodiment of the invention put on the wrist, Fig. 1b shows a tourniquet according to another embodiment put on the wrist, Fig 2a shows a block diagram of the embodiment of a system according to the invention, Fig. 2b shows a block diagram of an alternative embodiment of the system according to the invention, Fig. 2c shows a block diagram of yet another embodiment of the system according to the invention Fig. 3 shows a flowchart of a method according to the invention, and Fig. 4 shows the measurement of blood flow through a vessel using a plethysmograph.

### Description of the embodiments

First embodiment of the invention provides a bandage shown as a cross-section view in Fig. 1a, in the form of an arterial tourniquet, designed for attaching to the wrist, in a place where radial artery RA and ulnar artery UA are on the opposite sides of the wrist. Arterial tourniquet is provided with the tourniquet body 1 made of the transparent strip of flexible material wrapped around the wrist and provided with a first fastening element 3 and a second fastening element 3a, located on the opposite faces of the strip, from the outside and from the inside, respectively. Performing the tourniquet of transparent material allows easy inspection of the dressed place. The first and second fastening element 3 and 3a are made of an adhesive material, preferably a velcro strip. On the inner side the tourniquet 1 is provided with a first compression element, compressing the radial artery which is usually punctured during vascular surgeries. It is a balloon 2 filled with gas via a tubular connection on the side of the tourniquet, not shown. In the preferred embodiment, the tourniquet is provided with a second compression element, also in the form of a balloon 8.

Balloons 2 and 8 preferably have a shape of a spindle cushion with a thickness in a range of 0.5 - 1.5 cm, dimensions based on the order of few centimetres.

Alternatively, balloons filled with a liquid can be used, however, this is associated with a complication related to the need of providing a liquid reservoir and periodic leakage controls. Meanwhile, in the present application, a pneumatic solution is sufficient.

With the system according to the invention the tourniquet can also be used provided with compression elements other than pneumatic ones. They may include mechanical contracting and relaxing elements according to a control signal, clamping or stretching elements or actuators. An example of a system according to the invention with the CPU 210, a single actuator 22 and a blood loss sensor 5 and a blood flow sensor 26 is shown in Fig. 2a. In this configuration, the system allows, after putting on the tourniquet, to set the compression of actuator 25 controlling the bleeding at the site of bleeding, to the compression value corresponding to a pressure substantially higher than the patient's systolic pressure, and then gradually loosening until reaching blood flow. CPU obtains the information on the blood flow or lack of flow based on the signal from the flow sensor. Additionally, the use of the blood loss sensor allows protection against excessive loosening. The blood flow sensor may be a pressure gauge with changes detection circuit or even a microphone. It is important to receive the information that there is a blood flow through the vessel based on the threshold value of pressure changes or an acoustic pulse signal. As a result, if it stops appropriate steps can be taken quickly.

A plethysmograph attached to the digit can be also used as the flow measurement circuit. A block diagram of the system suitable for this configuration is shown in Fig. 2b. The use of the plethysmograph measuring the blood flow in the digit requires forcing the blood flow only through the damaged vessel, dressed with a tourniquet. Usually the vessel is a radial artery and the plethysmograph 26a is mounted on the digit of the palm. In this situation, the plethysmograph 26a reading corresponds to the actual flow through the radial artery only if the ulnar artery parallel to it is blocked, because plethysmograph placed on the digit measures the total and resultant blood flow through the two arteries: radial and ulnar. Therefore, in this embodiment, the system is provided with a second actuator 25b to bias the compression element to the second parallel vessel. Applying the compression corresponding to the pressure value exceeding the typical systolic pressure allows blocking the second parallel vessel for the duration of the flow measurement.

A block diagram of an automatic compression bandage system with a pneumatic system provided with a tourniquet shown in Fig. 1 is shown in Fig. 2c. The tourniquet is expanded with adapted CPU 210, which is the microcontroller provided with a memory 212, connected to peripheral devices 213, such as buttons, sensors and display devices 214. The power source is not shown in the drawing. The microcontroller is configured to process signals from the sensors and to control the operation of the pump 211 and valves 215, 233, 223.

The loss of blood sensor 5, returning a signal in the absence of homeostasis, i.e., a situation where blood is leaking from the dressed damaged vessel, is arranged inside the tourniquet connected to the CPU.

In addition, the system uses the blood flow sensor 226 connected to the CPU 210 via a filter converter 227 and analogue-digital converter 221. The blood flow sensor is an acoustic heart rate monitor. However, it can be replaced by the gauge. In this case, the detection of the blood flow is implemented by the software in the microcontroller by analyzing fluctuations in the pressure gauge. Another alternative is the use of a Doppler probe. Another preferred solution is use of a plethysmograph to measure the blood flow. This solution requires blocking of the blood flow in an ulnar artery parallel to the radial artery, as will be detailed below. A preferred solution is also the usual microphone. The blood flow may then be detected by analyzing the cyclic portions of recorded audio signal.

One of the ports of the CPU 210 is connected to the electric air pump 211. The outlet of the pump is connected to the first automatic diverter valve 215 via the pipe 211a, allowing air flow from the pump to the second automatic diverter valve 223, or the third automatic diverter valve 233, depending on the settings set at the control input. The control input of the first automatic diverter valve 215 is connected to the CPU 210.

The second automatic diverter valve 223 is connected to the first diverter valve 215, the first pressure sensor 225 and the deflation outlet 223a. The control input of the second automatic diverter valve 223 is connected to the CPU 210. Depending on the settings set at this input, the automatic diverter valve 223 connects the first pressure sensor 225 either with the pomp 211 using the first automatic diverter valve 215 or with the outlet 223a, designed for deflation. The first pressure sensor 225 is connected to the first balloon 2.

The third automatic diverter valve 233 is connected to the first diverter valve 215, the second pressure sensor 235 and the deflation outlet 223a. The control input of the third automatic diverter valve 233 is connected to the CPU 210. Depending on the settings set at this input, the automatic diverter valve 233 connects the second pressure sensor 235 either with the pomp 211 using the first automatic diverter valve 215 or with the outlet 233a, designed for deflation. The second pressure sensor 235 is connected to the second balloon 8.

Due to this configuration the first balloon 2 and the second balloon 8 can be pumped and deflated independently of each other, under the control of the CPU 210. Alternatively, instead of the pump 211, a cylinder with compressed gas, in particular air, may be used, making the system more portable. With the first balloon 2, the tourniquet body 1 houses the first sensor 226 of blood flow in the radial artery, which transmits the signal to the CPU 210 via the filter 227 and the analogue-digital converter 221.

With the second balloon 8, the tourniquet body 1 houses the second sensor 236 of blood flow in the ulnar artery, which transmits the signal to the CPU 210 via the filter 237 and the analogue-digital converter 231.

Filters 227 and 237 enable so-called smoothing the flow detection signal and the elimination of random fluctuations. They can be low-pass or band-pass filters.

Alternatively, digital sensors 236 may be used instead of analogue heart rate monitors 226 and filtration can be implemented by the software.

The system presented below allows dressing the ulnar artery damaged after the surgery and providing proper bandage compression using the first balloon 2. This compression corresponds to the initial pressure significantly greater than systolic blood pressure - for example, 200 mmHg. Unfortunately the compression greater than systolic pressure blocks the blood flow through the damaged radial artery. Literature shows that there is a range of pressures exerted by the compression element on the damaged vessel at the lesion site, which are lower than the systolic pressure but they stop the bleeding. As such a value the literature indicated mean arterial pressure (I/3*systolic blood pressure +2/3*value of diastolic pressure). This value is not confirmed by the experiment, setting it arbitrarily often leads to loss of blood from the vessel, what is unacceptable. The pressure range which ensures optimal conditions for healing, *i.e*., the blood flow through the damaged artery and no blood loss from the lesion site depends on the individual patient and progress of the healing process. Falling in this range is ensured by implementation of the method according to the invention. The flowchart for this process is shown in Fig. 3. After dressing the artery, automatic adjustment 30 starts. The pre-set pressure value in the balloon 2 is higher than the systolic blood pressure, for example 200 mmHg - stage 31. The pressure is reduced with the quantum dP in stage 32. Good effects are achieved due to quantum dP reduction equal to 5 mmHg, although the value may be adaptively adjusted during operation of the algorithm. This stage is repeated as long as the blood flow through the damaged vessel detected in stage 33 is detected. If the blood flow is detected, and there is no loss of blood checked in stage 37, or stop criterion 35 is not reached then the pressure control is suspended for the pressure drop time. The preferred pressure drop value is approx. 5 minutes. This value provides the flexibility of the system and monitoring of the artery status, in particular detecting the absence of flow, almost continuously, completely unreachable for medical staff performing these tasks manually. Sometimes it may be appropriate to extend this value up to a half an hour. This situation occurs when the patient's healing is progressing slowly and changes of the status are pointless. Sometimes, however, it is appropriate to shorten this time to even less than a minute. Therefore, more advanced embodiment of the invention proposes variation of the pressure drop value. Stop criterion 34 is to reduce the pressure below diastolic pressure. It is known then that dressing the vessel is no longer needed and the operation of the system can be completed - stage 35. It is, however, possible to use other stop criteria, such as the elapsed time since the beginning of the algorithm.

Proper operation requires absolute protection against the loss. Therefore, it is appropriate to carry out a stage 37 to check the blood flow at each pressure reduction and if detected to increase thinning until its cessation - stage 38. Alternatively, detection of blood loss can be performed in a continuous process and the mechanism of interrupting the operation of the CPU can be applied so that in case the blood loss is detected to immediately increase the pressure P in the balloon 2.

The situation in which there is no blood flow through the vessel and yet blood loss is detected is an unusual situation and may indicate an incorrect placement of the tourniquet or a malfunction.

Proper handling of such a situation requires intervention of a nurse or a doctor. Confirmation of such a situation completes the method according to the invention generating an alarm signal - stage 39.

When a plethysmograph is used as the blood flow sensor it is required to use the tourniquet in the embodiment shown in Fig. 1b. Examining the blood flow through the plethysmograph is shown in Fig. 4. The plethysmograph 4 is mounted on a digit. Ulnar artery UA at the time of detection is blocked. In the system according to the invention, this is achieved by setting the pressure within a tourniquet higher than the systolic blood pressure, in the balloon 8 disposed at the ulnar artery. Blood flow in the radial and ulnar artery takes place in parallel. Ulnar artery blockage makes the only flow that can be detected by the plethysmograph 4 a flow through the dressed radial artery. Thus, if the plethysmograph indicates no flow, it is possible to conclude that there is no flow in the damaged radial artery. However, if it detects the flow then it is the flow through the damaged radial artery.

Additionally, ulnar artery blockage is connected with increasing the flow through the radial artery, which is beneficial to the healing process. Partial blockage of an ulnar artery is enough, then it is preferred, in the time between successive blood flow detections, to set the blood pressure within the range between the values of systolic and diastolic pressure in the balloon 8. As a result, the blood flow of the radial artery is increased and the blood flow in the ulnar artery is not stopped.

An additional balloon 8 can also be used for a variant with no plethysmograph with another flow sensor. Then additionally the system according to the invention can be used to periodically increase the flow through the damaged artery by compressing a healthy ulnar artery.

Alternatively, the second balloon 8 can be used for a bandage, and the first balloon 2 for the pressure measurement and compression on a healthy vessel.

That means that the configuration is somewhat redundant. By resignation from the symmetry feature of the tourniquet's balloons and by use of the first balloon 2 only for the bandage, and the second balloon 2 only for the measurement of the patient's pressure and compression on a healthy vessel, the first heart rate monitor 201 can be eliminated. With additional automatic valves, the system can be reconfigured so as to use a common pressure sensor and read it sequentially, switching it between the first balloon 2 and the second balloon 8.

Alternatively, two air pumps can be used and the switched valve 215 can be abandoned. Any other digitally controlled compression methods can be used.

The system may be performed with the additional pressure sensor located elsewhere and connected electrically to the CPU 210, independent of compression on the arteries, *i.e*., the first balloon 2 and the second balloon 8.

Prior art discloses numerous other ways to apply compression corresponding to certain pressure values. Accordingly, the device described above with a pneumatic solution should be considered only as an example. The invention can be implemented by using mechanical compression and spring elements or those using threads and servos.

The above listed embodiments should be understood as illustrative and not limiting the invention. Most endovascular procedures are carried out through radial artery rather than the ulnar one. However, dressing with the system of the invention, and pressure control of the invention can also be achieved in the ulnar artery. It is also clear that the method and system of the invention can be applied not only to the wrist, but also in other parts of the patient's hand and on the other limbs, in particular to dress the femoral artery. A person skilled in automatic and manual methods of detecting the blood flow and dressing techniques of vascular damages resulting from intravascular procedures is able to realize the idea of the invention in numerous ways not quoted here.

## Claims

1. A tourniquet (1) provided with a controlled damaged blood vessel compression element (2) adapted to compress a damaged blood vessel at a lesion site, a blood flow sensor (26, 26a, 226) for detecting the blood flow through the compressed damaged blood vessel and a blood loss sensor (5) for detecting the blood flow from the compressed damaged blood vessel, **characterized in that** the said tourniquet (1) is provided further with a second healthy vessel compression element (8) configured to compress a healthy vessel and control the blood flow therein, said tourniquet being **characterized in that** the damaged blood vessel compression element (2) and the healthy vessel compression element (8) are controlled independently.

2. The tourniquet of claim 1, wherein it is further provided with a second blood flow sensor (26a, 236) for detecting the blood flow through the compressed healthy blood vessel.

3. An automatic compression bandage system provided with the tourniquet of claim 1 or 2, wherein it is provided with an interface for electronic pressure control of the compression elements (2, 8), and a CPU (210) provided with a memory and connected to a compression element interface.

4. The automatic compression bandage system according to claim 3, wherein the compression elements (2, 8) are balloons, whereas the system further comprises a pump (211) and automatic valves (223, 233) whose control input is connected to the CPU (210) and adapted to connect the balloons (2, 8) to the pump (211) and to empty them according to the setting of their control inputs.

5. The automatic compression bandage system according to one of claims 3 - 4, wherein the blood flow sensors (26, 26a, 226, 236) for detecting the blood flow through both compressed vessels and the blood loss sensor (5) for detecting the blood flow from the compressed vessel are located in the immediate vicinity of the compression elements (2, 8).

6. The automatic compression bandage system according to one of claims 3 - 5, wherein the blood flow sensor (26, 26a, 226, 236) is a heart rate monitor, whose output is connected to the input of the CPU (210) capable of detecting changes in a heart rate value.

7. The automatic compression bandage system according to one of claims 3-5 wherein the blood flow sensor (26, 26a, 226, 236) is a pressure sensor, whose output is connected to the input of the CPU (210) capable of detecting changes in a the pressure value.

8. The automatic compression bandage system according to one of claims 3 - 5, wherein the blood flow sensor (26, 26a, 226, 236) is a microphone, whose output is connected to the input of the CPU (210) capable of analysis of an audio signal.

9. The automatic compression bandage system according to one of claims 3 - 5, wherein the blood flow sensor (26, 26a, 226, 236) is a Doppler probe.

10. The automatic compression bandage system according to one of claims 3 - 5, wherein the blood flow sensor (26, 26a, 226, 236) is a plethysmograph, arranged downstream of the compression.

## Patentansprüche

1. Eine Aderpresse (1), die mit einem gesteuerten Kompressionselement (2) für beschädigte Blutgefäße versehen ist, das geeignet ist, ein beschädigtes Blutgefäß an einer Läsionsstelle zusammenzudrücken, einem Blutflusssensor (26, 26a, 226) zum Erfassen des Blutflusses durch das zusammengedrückte beschädigte Blutgefäß und einem Blutverlustsensor (5) zum Erfassen des Blutflusses aus dem zusammengedrückten beschädigten Blutgefäß, **dadurch gekennzeichnet, dass** die Aderpresse (1) ferner mit einem zweiten Kompressionselement (8) für gesunde Blutgefäße versehen ist, das so konfiguriert ist, dass es ein gesundes Blutgefäß komprimiert und den Blutfluss darin steuert, wobei die Aderpresse **dadurch gekennzeichnet ist, dass** das Kompressionselement (2) für beschädigte Blutgefäße und das Kompressionselement (8) für gesunde Blutgefäße unabhängig voneinander gesteuert werden.

2. Die Aderpresse nach Anspruch 1, wobei sie ferner mit einem zweiten Blutflusssensor (26a, 236) zum Erfassen des Blutflusses durch das komprimierte gesunde Blutgefäß versehen ist.

3. Ein automatisches Kompressionsverbandsystem, das mit der Aderpresse nach Anspruch 1 oder 2 versehen ist, wobei es mit einer Schnittstelle zur elektronischen Drucksteuerung der Kompressionselemente (2, 8) und einer CPU (210) versehen ist, die mit einem Speicher versehen und mit einer Schnittstelle für Kompressionselemente verbunden ist.

4. Das automatische Kompressionsverbandsystem nach Anspruch 3, bei dem die Kompressionselemente (2, 8) Ballons sind, wobei das System ferner eine Pumpe (211) und automatische Ventile (223, 233) aufweist, deren Steuereingang mit der CPU (210) verbunden ist und die geeignet sind, die Ballons (2, 8) mit der Pumpe (211) zu verbinden und sie entsprechend der Einstellung ihrer Steuereingänge zu entleeren.

5. Das automatische Kompressionsverbandsystem nach einem der Ansprüche 3-4, wobei die Blutflusssensoren (26, 26a, 226, 236) zum Erfassen des Blutflusses durch beide komprimierten Gefäße und der Blutverlustsensor (5) zum Erfassen des Blutflusses aus dem komprimierten Gefäß in unmittelbarer Nähe der Kompressionselemente (2, 8) angeordnet sind.

6. Das automatische Kompressionsverbandsystem nach einem der Ansprüche 3-5, wobei der Blutflusssensor (26, 26a, 226, 236) ein Herzfrequenzmonitor ist, dessen Ausgang mit dem Eingang der CPU (210) verbunden ist, die in der Lage ist, Änderungen in einem Herzfrequenzwert zu erfassen.

7. Das automatische Kompressionsverbandsystem nach einem der Ansprüche 3-5, wobei der Blutflusssensor (26, 26a, 226, 236) ein Drucksensor ist, dessen Ausgang mit dem Eingang der CPU (210) verbunden ist, die in der Lage ist, Änderungen in einem Druckwert zu erfassen.

8. Das automatische Kompressionsverbandsystem nach einem der Ansprüche 3-5, wobei der Blutflusssensor (26, 26a, 226, 236) ein Mikrofon ist, dessen Ausgang mit dem Eingang der CPU (210) verbunden ist, die in der Lage ist, ein Audiosignal zu analysieren.

9. Das automatische Kompressionsverbandsystem nach einem der Ansprüche 3-5, wobei der Blutflusssensor (26, 26a, 226, 236) eine Dopplersonde ist.

10. Das automatische Kompressionsverbandsystem nach einem der Ansprüche 3-5, wobei der Blutströmungssensor (26, 26a, 226, 236) ein Plethysmograph ist, der stromabwärts der Kompression angeordnet ist.

## Revendications

1. Tourniquet (1) pourvu d'un élément de compression de vaisseau sanguin endommagé contrôlé (2) adapté pour comprimer un vaisseau sanguin endommagé au niveau d'un site de lésion, un capteur de débit sanguin (26, 26a, 226) pour détecter le débit sanguin à travers le vaisseau sanguin comprimé endommagé et un capteur de perte de sang (5) pour détecter le débit sanguin provenant du vaisseau sanguin comprimé endommagé, **caractérisé en ce que** ledit tourniquet (1) est pourvu en outre d'un second élément de compression de vaisseau sain (8) configuré pour comprimer un vaisseau sain et contrôler le débit à l'intérieur, ledit tourniquet étant **caractérisé en ce que** l'élément de compression de vaisseau sanguin endommagé (2) et l'élément de compression de vaisseau sain (8) sont contrôlés indépendamment.

2. Tourniquet selon la revendication 1, où il est en outre pourvu d'un deuxième capteur de débit sanguin (26a, 236) pour détecter le débit sanguin à travers le vaisseau sanguin sain comprimé.

3. Système de bandage à compression automatique pourvu du tourniquet selon la revendication 1 ou 2, où il est pourvu d'une interface pour le contrôle électronique de la pression des éléments de compression (2,8), et d'un UCT (210) pourvue d'une mémoire et connectée à une interface d'élément de compression.

4. Système de bandage à compression automatique selon la revendication 3, où les éléments de compression (2,8) sont des ballons, tandis que le système comprend en outre une pompe (211) et des vannes automatiques (223, 233) dont l'entrée de contrôle est connectée à l'UCT (210) et adaptée pour connecter les ballons (2,8) à la pompe (211) et pour les vider selon le réglage de leurs entrées de contrôle.

5. Système de bandage à compression automatique selon l'une des revendications 3 à 4, où les capteurs de débit sanguin (26, 26a, 226, 236) pour détecter le débit sanguin à travers les deux vaisseaux comprimés et le capteur de perte de sang (5) pour détecter le débit sanguin à partir du vaisseau comprimé sont situés à proximité immédiate des éléments de compression (2, 8).

6. Système de bandage à compression automatique selon l'une des revendications 3 à 5, où le capteur de débit sanguin (26, 26a, 226, 236) est un moniteur de fréquence cardiaque, dont la sortie est connectée à l'entrée de l'UCT (210) capable de détecter des changements dans une valeur de fréquence cardiaque.

7. Système de bandage à compression automatique selon l'une des revendications 3 à 5, où le capteur de débit sanguin (26, 26a, 226, 236) est un capteur de pression, dont la sortie est connectée à l'entrée de l'UCT (210) capable de détecter des changements dans une valeur de pression.

8. Système de bandage à compression automatique selon l'une des revendications 3 à 5, où le capteur de débit sanguin (26, 26a, 226, 236) est un microphone, dont la sortie est connectée à l'entrée de l'UCT (210) capable d'analyser un signal audio.

9. Système de bandage à compression automatique selon l'une des revendications 3 à 5, où le capteur de débit sanguin (26, 26a, 226, 236) est une sonde Doppler.

10. Système de bandage à compression automatique selon l'une des revendications 3 à 5, où le capteur de débit sanguin (26, 26a, 226, 236) est un pléthysmographe, disposé en aval de la compression.
